# EUROPEAN PATENT APPLICATION

(11) **EP 4 520 360 A1**
(43) Date of publication of application: **12.03.2025**
(21) Application number: 23831390.2
(22) Date of filing: 26.06.2023
(51) Int. Cl.: A61M 1/16

(54) **BLOOD PURIFICATION DEVICE**

(30) Priority: 28.06.2022 JP 2022103780
(71) Applicant: Nikkiso Co., Ltd., Tokyo 150-6022 (JP)
(72) Inventor: ISHIDA, Makoto, Makinohara-shi Shizuoka 421-0496 (JP)
(74) Representative: von Hirschhausen, Helge
(86) International application number: PCT/JP2023/023625
(87) International publication number: WO 2024/004950

(57) **Abstract**

A blood purification device (1) for purifying blood (L4) with the use of a dialysate (L3), said blood purification device (1) comprising a dialysis machine body (2), which is the main body of the device, and an RO unit (3) which is mounted to the dialysis machine body (2), wherein: the dialysis machine body (2) has a first group of parts (27) requiring maintenance and a mounting space (206) in which the first group of parts (27) is exposed; and, when the RO unit (3) is dismounted from the dialysis machine body (2), the mounting space (206) is open to the outside of the dialysis machine body (2), and when the RO unit (3) is mounted to the dialysis machine body (2), the mounting space (206) is closed by the RO unit (3).

## Description

### TECHNICAL FIELD

The present invention relates to a blood purification device.

### BACKGROUND OF THE INVENTION

As a conventional technique, a hemodialysis system is known, in which a blood purification device which purifies blood is coupled to a dialysate generator which generates dialysis water (see, e.g., Patent Literature 1).

This conventional hemodialysis system has an openable back panel on the back side of the dialysate generator and is configured such that this back panel is opened to allow for replacement of carbon filter and reverse osmosis.

### CITATION LIST

### PATENT LITERATURES

Patent Literature 1: US Patent Application Publication No. 2021-0128807, Specification

### SUMMARY OF THE INVENTION

In the blood purification device of this conventional hemodialysis system, components that require regular replacement and maintenance are located not only on the front side but also on the back side facing the dialysate generator. However, the problem is that the structure does not take into account maintainability of these components.

Therefore, it is an object of the invention to provide a blood purification device with improved maintainability.

To achieve the object above, the invention provides a blood purification device that purifies blood using dialysate, the blood purification device comprising:
a first unit that is a main body of the device; and
a second unit to be attached to the first unit,
wherein the first unit comprises a first group of components requiring maintenance, and an attachment space in which the first group of components is exposed,
wherein the attachment space is open to the outside of the first unit in a state that the second unit is detached from the first unit, and
wherein the attachment space is closed by the second unit in a state that the second unit is attached to the first unit.

### Advantageous Effects of the Invention

According to the invention, it is possible to improve maintainability.

### BRIEF DESCRIPTION OF DRAWINGS

Fig. 1A is an example block diagram illustrating a blood purification device.
Fig. 1B is an explanatory diagram illustrating an example of connection of tubes., etc. between a dialysis machine main body and a RO unit.
Fig. 1C is an explanatory diagram illustrating an example of flows of dialysate and blood, etc. in the dialysis machine main body.
Fig. 2A is a schematic diagram illustrating an example of a state in which the dialysis machine main body and the RO unit are integrated.
Fig. 2B is a schematic diagram illustrating an example of a state in which the RO unit is detached from the dialysis machine main body.
Fig. 3A is a diagram illustrating an example of a state in which a backside cover is attached.
Fig. 3B is a diagram illustrating an example of a state in which a replacement cover is detached.
Fig. 3C is a diagram illustrating an example of a state in which an upper cover is further detached.
Fig. 4A is a diagram illustrating an example of a state in which the RO unit is detached from the dialysis machine main body.
Fig. 4B is a perspective view showing an example of the state in which the RO unit is detached from the dialysis machine main body.
Fig. 5A is a diagram illustrating an example of a first surface side of the RO unit.
Fig. 5B is a diagram illustrating an example of a second surface side.
Fig. 5C is a diagram illustrating an example of a side surface side.

### DETAILED DESCRIPTION OF THE INVENTION

### Embodiment

### General configuration of Blood purifying device 1

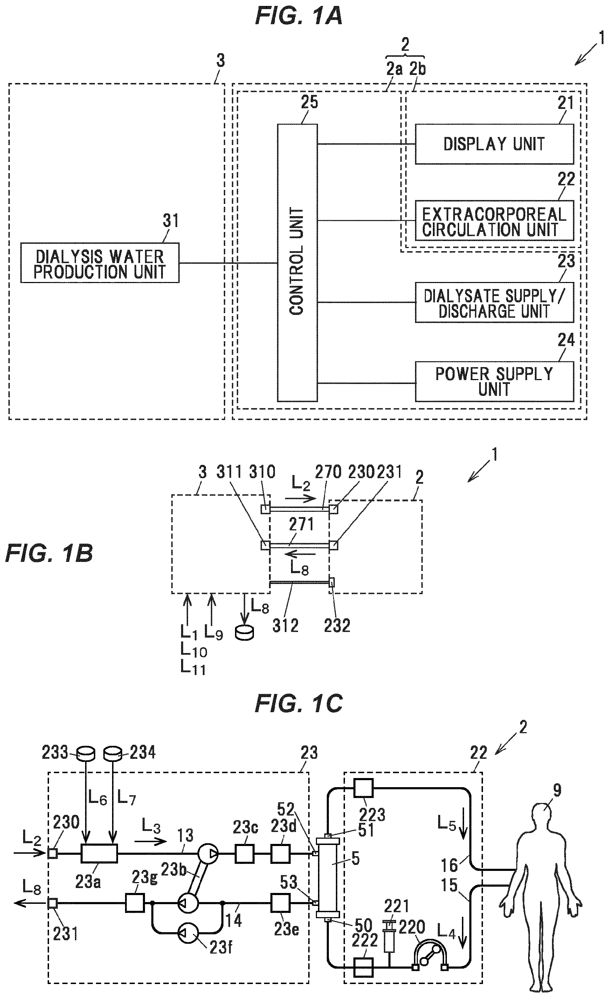

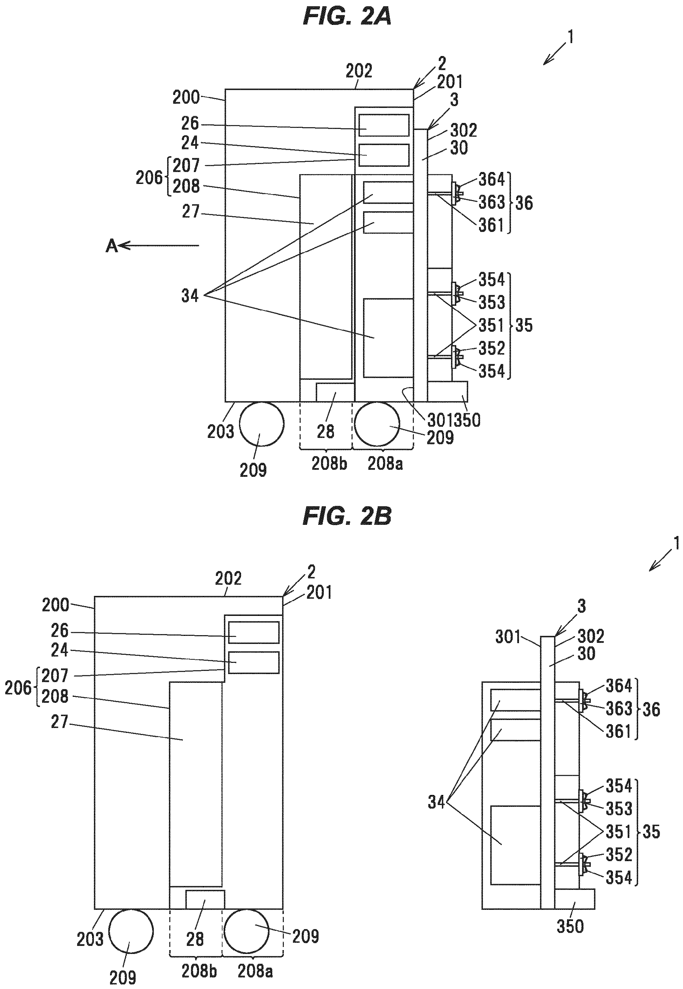

Fig. 1A is an example block diagram illustrating a blood purification device in an embodiment, Fig. 1B is an explanatory diagram illustrating an example of connection of tubes., etc. between a dialysis machine main body and a RO unit, and Fig. 1C is an explanatory diagram illustrating an example of flows of dialysate and blood, etc. in the dialysis machine main body. Fig. 2A is a schematic diagram illustrating an example of a state in which the dialysis machine main body and the RO unit in the embodiment are integrated, and Fig. 2B is a schematic diagram illustrating an example of a state in which the RO unit is detached from the dialysis machine main body. In the drawings of the embodiment described below, a scale ratio or shape may be different between the drawings or different from an actual ratio or shape. In addition, in Fig. 1B, flows of liquids such as dialysate L₃ are indicated by arrows.

As shown in Figs. 1A to 1C, a blood purification device 1 in the present embodiment is a medical device that performs dialysis treatment in which blood L₄ of a patient 9 is purified using a dialyzer 5 as a blood purifier. Purified blood L₅ shown in Fig. 1C indicates blood that is returned to the patient 9 after purification of the blood L₄.

The blood purification device 1 purifies the blood L₄ using the dialysate L₃. The blood purification device 1 has a dialysis machine main body 2 as the first unit that is a main body of the device, and a RO unit 3 as the second unit to be attached to the dialysis machine main body 2. The dialysis machine main body 2 has a first group of components 27 requiring maintenance, and an attachment space 206 in which the first group of components 27 is exposed. The attachment space 206 is open to the outside of the dialysis machine main body 2 in a state that the RO unit 3 is detached from the dialysis machine main body 2, and the attachment space 206 is closed by the RO unit 3 in a state that the RO unit 3 is attached to the dialysis machine main body 2.

As shown in Figs. 1A to 2B, the blood purification device 1 is generally configured such that the dialysis machine main body 2 has a dialysate supply/discharge unit 23 as a dialysate production unit that produces the dialysate L₃ by mixing dialysis water L₂ with dialysate concentrates and introduces and withdraws the dialysate L₃ to and from the dialyzer 5 that purifies blood of a patient, and a first attachment portion 29 (described later), the first group of components 27 requiring maintenance for at least one of production and introduction/withdrawal of the dialysate L₃ is exposed in the attachment space 206, the RO unit 3 has a second attachment portion 300 (described later) to be attached to and detached from the first attachment portion 29 in a manner capable of closing the attachment space 206 when attaching the RO unit 3 to the dialysis machine main body 2 and exposing the attachment space 206 when detaching the RO unit 3, a liquid supply port 37 through which external raw water L₁ is supplied, and a dialysis water production unit 31 to produce the dialysis water L₂ by removing impurities from the raw water L₁ supplied through the liquid supply port 37, and the produced dialysis water L₂ is supplied to the first unit.

The dialysis water L₂ is RO (Reverse Osmosis) water obtained by removing bacteria and impurities from the raw water L₁ introduced from the outside. The dialysate concentrates are an undiluted B-solution L₆ and an undiluted A-solution L₇.

### Configuration of Dialysis machine main body 2

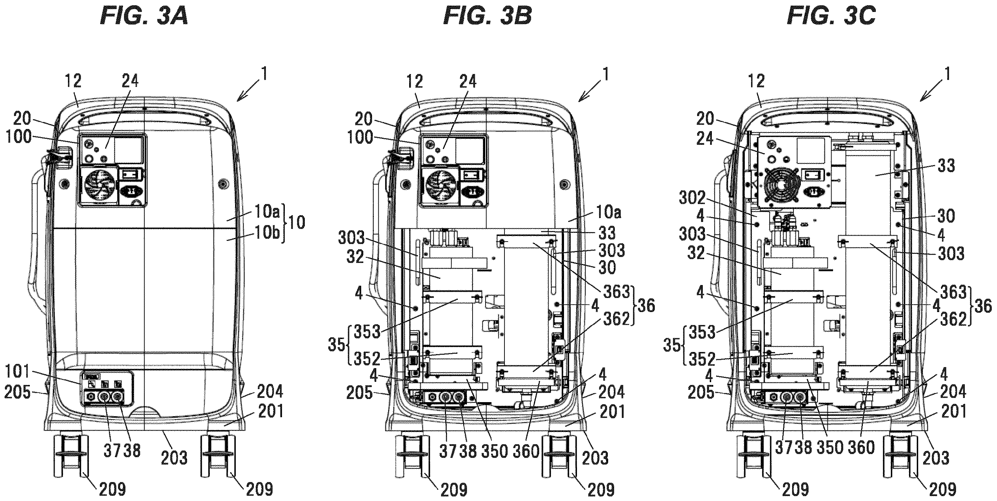

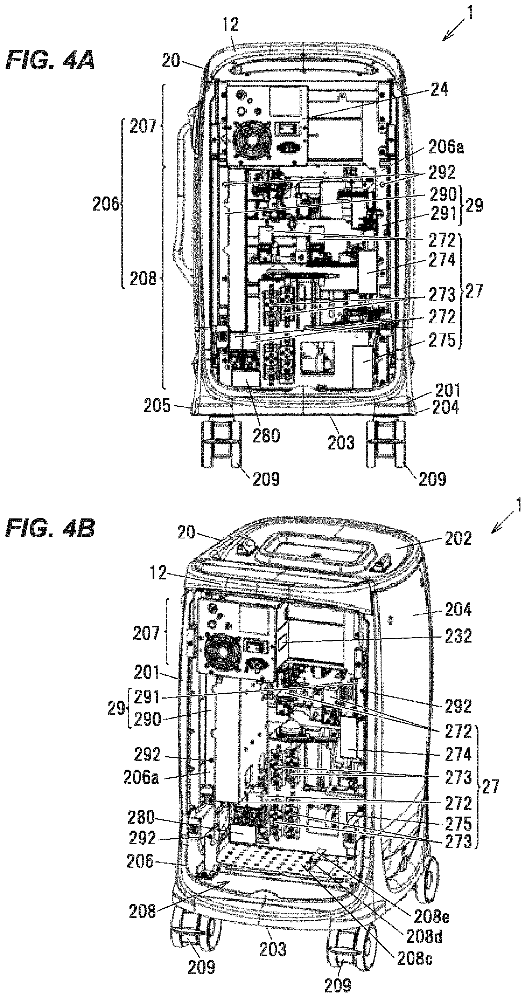

Fig. 3A is a diagram illustrating an example of a state in which a backside cover is attached to the blood purification device in the present embodiment, Fig. 3B is a diagram illustrating an example of a state in which a replacement cover is detached, and Fig. 3C is a diagram illustrating an example of a state in which an upper cover is further detached. Fig. 4A is a diagram illustrating an example of a state in which the RO unit is detached from the dialysis machine main body in the present embodiment, and Fig. 4B is a perspective view showing an example of the state in which the RO unit is detached from the dialysis machine main body.

As shown in Fig. 1A, the dialysis machine main body 2 is generally composed of a display unit 21, an extracorporeal circulation unit 22, the dialysate supply/discharge unit 23, a power supply unit 24, and a control unit 25. As an example, the dialysis machine main body 2 is further divided into a lower unit 2a having the dialysate supply/discharge unit 23, the power supply unit 24 and the control unit 25, and an upper unit 2b having the display unit 21 and the extracorporeal circulation unit 22, as shown in Fig. 1A. The upper unit 2b is arranged, e.g., on an upper surface 202 of a housing 20. The dialysis machine main body 2 may be configured such that the lower unit 2a and the upper unit 2b are integrated into one unit.

As an example, the display unit 21 is a touch panel that displays the progress, etc. of treatment and receives touch operations by a user.

As shown in Fig. 1C, the extracorporeal circulation unit 22 includes a blood pump 220, a syringe pump 221, a flowmeter 222, and a flowmeter 223.

The blood pump 220 is, e.g., a peristaltic pump. The blood pump 220 is configured to send the blood L₄ in an arterial blood circuit 15 to the dialyzer 5.

The syringe pump 221 is, e.g., a syringe-shaped pump. The syringe pump 221 is configured to introduce an anticoagulant, which suppresses coagulation of the blood L₄, into the arterial blood circuit 15.

The flowmeter 222 and the flowmeter 223 are, e.g., flow sensors using ultrasonic waves or laser light. The flowmeter 222 measures a flow rate of the blood L₄ flowing through the arterial blood circuit 15. The flowmeter 223 is arranged on a venous blood circuit 16 and measures a flow rate of the purified blood L₅ flowing through the venous blood circuit 16.

As shown in Fig. 1C, the dialysate supply/discharge unit 23 includes a dialysate dilution unit 23a, a duplex pump 23b, a pressure gauge 23c, a flowmeter 23d, a flowmeter 23e, a water removal pump 23f, and a pressure gauge 23g.

As shown in Fig. 1C, the dialysate dilution unit 23a is generally configured to create the dialysate L₃ by using the dialysis water L₂ supplied from the RO unit 3, the undiluted B-solution L₆ supplied from an undiluted B-solution tank 233 and the undiluted A-solution L₇ supplied from an undiluted A-solution tank 234, and adjusting a concentration of a chemical liquid. The undiluted B-solution tank 233 and the undiluted A-solution tank 234 are connected to the dialysis machine main body 2, but are not limited thereto and may be configured to be arranged inside the dialysis machine main body 2.

As shown in Fig. 1C, the duplex pump 23b is arranged over a dialysate introduction line 13 and a dialysate discharge line 14. The duplex pump 23b is configured to apply pressure to the dialysate introduction line 13 to send the dialysate L₃ to the dialyzer 5 and to apply pressure to the dialysate discharge line 14 to send a dialysate effluent L₈ to the RO unit 3 through an outlet tube 231 and a tube 271. The duplex pump 23b sends the dialysate L₃ and the dialysate effluent L₈ at the same pressure in the dialysate introduction line 13 and the dialysate discharge line 14. The dialysate effluent L₈ may be sent to an effluent tank connected to the dialysis machine main body 2.

The pressure gauge 23c and the pressure gauge 23g are, e.g., semiconductor pressure sensors using diaphragms. The pressure gauge 23c measures pressure of the dialysate L₃. The pressure gauge 23g measures pressure of the dialysate effluent L₈.

The flowmeter 23d and the flowmeter 23e are, e.g., flow sensors using ultrasonic waves or laser light. The flowmeter 23d measures a flow rate of the dialysate L₃. The flowmeter 23e measures a flow rate of the dialysate effluent L₈.

The water removal pump 23f is connected so as to bypass the duplex pump 23b. The water removal pump 23f is provided to remove water from the blood L₄ of the patient 9 flowing through the dialyzer 5.

The dialyzer 5 includes a blood introduction port 50, a blood discharge port 51, a dialysate introduction port 52, and a dialysate discharge port 53. As shown in Fig. 1C, the dialyzer 5 is arranged such that the dialysate introduction port 52 is connected to the dialysate introduction line 13 and the dialysate discharge port 53 is connected to the dialysate discharge line 14. The dialyzer 5 is arranged also such that the blood introduction port 50 is connected to the arterial blood circuit 15 and the blood discharge port 51 is connected to the venous blood circuit 16.

The dialyzer 5 has plural hollow fibers and is configured to purify the blood L₄ by the hollow fibers. A blood flow route connecting the blood introduction port 50 and the blood discharge port 51 through a blood purification membrane and a dialysate flow route connecting the dialysate introduction port 52 and the dialysate discharge port 53 are also formed in the dialyzer 5. The hollow fibers constituting the blood purification membrane form a hollow fiber membrane with microscopic holes penetrating from an outer peripheral surface to an inner peripheral surface, and impurities, etc., in the blood are transferred into the dialysate L₃ through the hollow fiber membrane.

As shown in Figs. 2A, 2B, 4A and 4B, in the dialysis machine main body 2, the attachment space 206 is composed of an opening 206a which is open on a back surface 201 of the housing 20, an upper space 207, and a lower space, the power supply unit 24 and an electronic circuit unit 26 are arranged in the upper space 207, and the first group of components 27 is exposed at an inner side in the lower portion serving as a maintenance space 208. When the RO unit 3 is attached to the dialysis machine main body 2, a second group of components 34 is located at an outer side of the maintenance space 208. As shown in Figs. 2A and 2B, the outer side of the maintenance space 208 is a first region 208a where the RO unit 3 fits, as an example. Then, the inner side of the maintenance space 208 is a second region 208b in which the first group of components 27 is arranged, as an example.

The power supply unit 24 is configured to supply power for the dialysis machine main body 2 and the RO unit 3 to operate. The electronic circuit unit 26 has the control unit 25. The control unit 25 is a microcomputer composed of a CPU (Central Processing Unit) performing calculation and processing, etc., of the acquired data according to a stored program, and a RAM (Random Access Memory) and a ROM (Read Only Memory) as semiconductor memories, etc. The control unit 25 is configured to comprehensively control the dialysis machine main body 2 and the RO unit 3.

As shown in Fig. 2A, the dialysis machine main body 2 has a front surface 200 and the back surface 201 that is a surface opposite to the front surface 200. When a main movement direction of the dialysis machine main body 2 is a direction in which a user moves it by pushing the back surface 201 side, the RO unit 3 is attached to the dialysis machine main body 2 with the attachment space 206 located on the back surface 201 side. The main movement direction is the direction of arrow A shown in Fig. 2A, and is a direction in which the user holding a handle 12 provided on the housing 20 pushes.

As shown in Fig. 2A, the dialysis machine main body 2 has four casters 209 on a lower surface 203 of the housing 20. The dialysis machine main body 2 can also move in a direction opposite to the direction of arrow A, and furthermore, can freely change directions and move since the casters 209 rotate about two intersecting rotation axes.

In the blood purification device 1, when the RO unit 3 is attached to the dialysis machine main body 2, the second group of components 34 is located below the power supply unit 24 of the dialysis machine main body 2, as shown in Fig. 2A. Therefore, a change in the center of gravity of the blood purification device 1 with or without the RO unit 3 is smaller than when this configuration is not adopted, and it is possible to ensure mobility and also suppress falling over regardless of with or without the RO unit 3.

As shown in Fig. 3A, the dialysis machine main body 2 also has a backside cover 10 that is located on the back surface 201 side and covers the attached RO unit 3. As shown in Figs. 3A and 3B, the backside cover 10 has an upper cover 10a attached at an upper portion of the attachment space 206, and a replacement cover 10b attached at a lower portion of the attachment space 206. The upper cover 10a has an upper opening 100 through which the power supply unit 24 is exposed. The replacement cover 10b has a lower opening 101 through which the liquid supply port 37 and a liquid discharge port 38 are exposed. The replacement cover 10b is removed to expose a carbon filter 32 and a reverse osmosis membrane 33 (described below) to allow replacement thereof.

As shown in Figs. 2A and 2B, the dialysis machine main body 2 has a liquid supply unit 28 that is located in the attachment space 206 and is capable of receiving dialysis water other than the dialysis water L₂ produced by the RO unit 3. This dialysis water other than the dialysis water L₂ is external dialysis water L₉ shown in Fig. 1B. In the event that, e.g., the RO unit 3 fails, the blood purification device 1 can perform dialysis treatment using the external dialysis water L₉ produced externally and supplied through the liquid supply unit 28.

### First group of components 27

The blood purification device 1 is configured so that the tubes, etc., of the arterial blood circuit 15 and the venous blood circuit 16, through which blood circulates, are replaced after treatment ends. This replacement is easy since the tubes, etc. are exposed to the outside.

In addition, the dialysis machine main body 2 is configured such that the tubes, etc. to circulate the dialysate L₃, the dialysate effluent L₈, cleaning water L₁₀, disinfectant L₁₁, etc. are arranged thereinside and are not exposed to the outside. The dialysis machine main body 2 is configured such that components requiring replacement or maintenance are accessible from the front surface 200, a side surface 204 and a side surface 205 of the housing 20. Furthermore, components which require replacement or maintenance and are located on the rear surface 201 side are accessible through the attachment space 206.

The first group of components 27 includes plural components necessary for at least one of production of the dialysate L₃, introduction of the dialysate L₃ and discharge of the used dialysate L₃, i.e., the dialysate effluent L₈. The first group of components 27 is accessible through the maintenance space 208 of the attachment space 206. The first group of components 27 includes, e.g., tube, conductivity cell, temperature sensor, flow rate sensor, pressure sensor, electromagnetic valve, pump, and filter. In Figs. 4A and 4B, plural electromagnetic valves 272, plural conductivity cells 273, a liquid feed pump 274 and a liquid discharge pump 275 are shown as the first group of components 27, as an example. The conductivity cells 273 measure the conductivity of the dialysate L₃ (the dialysate concentrate) during the dialysate production process. The liquid feed pump 274 is a pump to send the dialysate L₃ to the dialyzer 5. The liquid discharge pump 275 is a pump to discharge the dialysate effluent L₈ from the dialyzer 5.

### Positioning of RO unit 3

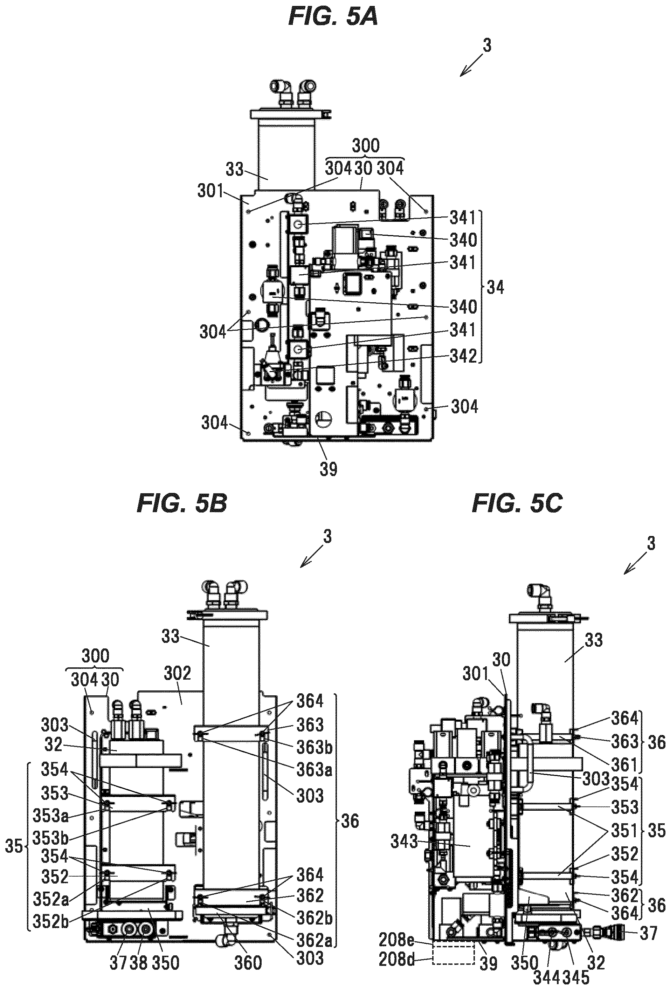

Fig. 5A is a diagram illustrating an example of a first surface side of the RO unit in the embodiment, Fig. 5B is a diagram illustrating an example of a second surface side of the RO unit, and Fig. 5C is a diagram illustrating an example of a side surface side of the RO unit. The tubes are not shown in Figs. 5A to 5C.

In the maintenance space 208, a base 208d is arranged on a lower surface 208c, as shown in Fig. 4B. The base 208d is formed by bending a metal plate and has an L-shape, as an example. A lower surface 39 of the RO unit 3 is placed on an upper surface 208e of the base 208d, as shown in Fig. 5C. That is, the upper surface 208e determines the position of the RO unit 3 in the vertical direction.

In the maintenance space 208, the first attachment portion 29 is provided on the side surface 204 side and on the side surface 205 side, as shown in Figs. 4A and 4B. The first attachment portion 29 is generally composed of an attachment plate 290 and an attachment plate 291 that have a plate shape. The attachment plate 290 and the attachment plate 291 have plural fastening holes 292. This fastening hole 292 is threaded and is configured so that a bolt 4 for attachment of the RO unit 3 is fastened therein. That is, the attachment plate 290 and the attachment plate 291 determine the position of the RO unit 3 in the front-rear direction.

### Configuration of RO unit 3

As shown in Figs. 5A to 5C, the RO unit 3 has a base plate 30. The base plate 30 is, e.g., made of a metal material and has a long plate shape. The base plate 30 has a first surface 301 facing the dialysis machine main body 2 and a second surface 302 opposite to the first surface 301. The second group of components 34 requiring maintenance is arranged on the first surface 301. The carbon filter 32 and the reverse osmosis membrane 33, which need to be replaced regularly, are arranged on the second surface 302.

In addition, the base plate 30 has, on the second surface 302, a first support portion 35 supporting the carbon filter 32, and a second support portion 36 supporting the reverse osmosis membrane 33. The first support portion 35 and the second support portion 36 are configured such that the carbon filter 32 and the reverse osmosis membrane 33 can be attached and detached using thumbscrews 354 that do not require any tools.

Furthermore, the base plate 30 has plural through-holes 304 at the left and right ends, as shown in Figs. 5A and 5B. The second attachment portion 300 is composed of the base plate 30 and the plural through-holes 304. The through-holes 304 are formed so as to correspond to the fastening holes 292 of the dialysis machine main body 2. The RO unit 3 is attached to the dialysis machine main body 2 by tightening the bolts 4 in the fastening holes 292 through the through holes 304.

The first support portion 35 is composed of a support base 350, plural male screws 351, a lower support plate 352, an upper support plate 353, and plural thumbscrews 354.

The support base 350 is a stand to place the carbon filter 32 and is attached to the base plate 30. The male screws 351 are arranged, two for each of the lower support plate 352 and the upper support plate 353, and are attached to the base plate 30.

The lower support plate 352 and the upper support plate 353 are, e.g., made of a metal material and have a plate shape. As shown in Fig. 5B, the lower support plate 352 has a notch 352a and a notch 352b that are formed by cutting upward from the underside. Likewise, the upper support plate 353 has a notch 353a and a notch 353b that are formed by cutting upward from the underside.

The male screws 351 are inserted into the notch 352a, the notch 352b, the notch 353a and the notch 353b. The carbon filter 32, which is placed on the support base 350 and arranged between the male screws 351, is attached to the base plate 30 by fastening the thumbscrews 354 to the male screws 351. The user can detach the carbon filter 32 from the base plate 30 by rotating and loosening the thumbscrews 354 by hand and rotating the lower support plate 352 and the upper support plate 353 upward.

The second support portion 36 is composed of a support base 360, plural male screws 361, a lower support plate 362, an upper support plate 363, and plural thumbscrews 364.

The support base 360 is a stand to place the reverse osmosis membrane 33 and is attached to the base plate 30. The male screws 361 are arranged, two for each of the lower support plate 362 and the upper support plate 363, and are attached to the base plate 30.

The lower support plate 362 and the upper support plate 363 are, e.g., made of a metal material and have a plate shape. As shown in Fig. 5B, the lower support plate 362 has a notch 362a and a notch 362b that are formed by cutting upward from the underside. Likewise, the upper support plate 363 has a notch 363a and a notch 363b that are formed by cutting upward from the underside.

The male screws 361 are inserted into the notch 362a, the notch 362b, the notch 363a and the notch 363b. The reverse osmosis membrane 33, which is placed on the support base 360 and arranged between the male screws 361, is attached to the base plate 30 by fastening the thumbscrews 364 to the male screws 361. The user can detach the reverse osmosis membrane 33 from the base plate 30 by rotating and loosening the thumbscrews 364 by hand and rotating the lower support plate 362 and the upper support plate 363 upward.

The base plate 30 is also provided with handles 303 on the left and right ends of the second surface 302, as shown in Figs. 5B and 5C. The user can easily attach and detach the RO unit 3 by using the handles 303.

As shown in Fig. 5B, the RO unit 3 has the liquid supply port 37 and the liquid discharge port 38 at the bottom. A tube is connected to the liquid supply port 37 and the raw water L₁ is supplied to the RO unit 3 through the liquid supply port 37. A tube is connected to the liquid discharge port 38 and the dialysate effluent L₈ is discharged from the RO unit 3 through the liquid discharge port 38.

### Second group of components 34

The second group of components 34 includes plural components for, e.g., drawing in the raw water L₁ and producing and sending out the dialysis water L₂. The second group of components 34 includes, e.g., tube, pump, electromagnetic valve, conductivity cell, actuator, and filter. The filter is an endotoxin retentive filter that captures bacteria and endotoxins, or a fine particle filter to filter out fine particles. In Figs. 5A to 5C, plural electromagnetic valves 340, plural conductivity cells 341, a pressure reducing valve 342, an RO pump 343, a protection-side water supply pressure sensor 344 and a control-side water supply pressure sensor 345 are shown as the second group of components 34.

The conductivity cells 341 measure the conductivity of the dialysis water L₂ on the downstream side of the reverse osmosis membrane 33. The pressure reducing valve 342 reduces pressure applied to the dialysate tubing on the downstream side of the RO pump 343 and keeps the pressure constant. The RO pump 343 injects the raw water L₁, which has passed through the carbon filter 32, into the reverse osmosis membrane 33. The pressure of the RO pump 343 is adjusted in advance to be appropriate for the flow rate passing through the reverse osmosis membrane 33. The protection-side water supply pressure sensor 344 and the control-side water supply pressure sensor 345 measure pressure of the supply liquid. When there is a large difference between the pressures measured by the protection-side water supply pressure sensor 344 and the control-side water supply pressure sensor 345, the blood purification device 1 determines that a failure has occurred. In addition, when the water supply pressure falls to not more than a predetermined value, the blood purification device 1 controls and closes a water supply electromagnetic valve to prevent backflow.

The second group of components 34 is exposed on the first surface 301 of the RO unit 3, which facilitates maintenance.

### Attachment of RO unit 3

As shown in Fig. 1B, the dialysis machine main body 2 has an inlet tube 230, the outlet tube 231, and a power plug 232. The RO unit 3 has an outlet tube 310, an inlet tube 311, and a power cord 312.

When attaching the RO unit 3 to the dialysis machine main body 2, first, the inlet tube 230 is connected to the outlet tube 310 by a tube 270, the outlet tube 231 is connected to the inlet tube 311 by the tube 271, and the power cord 312 is connected to the power plug 232 provided on a side surface of the power supply unit 24. As an example, the inlet tube 230 and the outlet tube 231 are internally arranged in an inlet/outlet region 280 at the bottom of the attachment space 206, as shown in Figs. 4A and 4B.

The user can attach the RO unit 3 to the dialysis machine main body 2 by placing the lower surface 39 of the RO unit 3 on the upper surface 208e of the base 208d, aligning the first attachment portion 29 with the second attachment portion 300, and inserting and tightening the bolts 4 in the fastening holes 292 through the through-holes 304.

### Effects of the embodiment

The blood purification device 1 in the present embodiment can improve maintainability. In particular, in the blood purification device 1, the first group of components 27 requiring maintenance is exposed in the attachment space 206. This makes maintenance easier than when not exposed since the first group of components 27 is located within reach of the user's hand.

In the blood purification device 1, the carbon filter 32 and the reverse osmosis membrane 33, which require regular replacement, are arranged not on the first surface 301 facing the dialysis machine main body 2 but on the second surface 302 which is the opposite surface. This makes replacement easy even without detaching the RO unit 3 from the dialysis machine main body 2, as compared to the case where this configuration is not adopted. In addition, in the blood purification device 1, the second group of components 34 requiring maintenance is arranged on the first surface 301. This makes it easy to perform maintenance on even components that do not require replacement or maintenance as frequently as the carbon filter 32 and the reverse osmosis membrane 33.

In the blood purification device 1, the first support portion 35 supporting the carbon filter 32 and the second support portion 36 supporting the reverse osmosis membrane 33 are attached using the thumbscrews 354. Therefore, it is possible to easily detach and attach without using tools, as compared to the case where a tool is used for attachment.

In the blood purification device 1, the power supply unit 24 and the electronic circuit unit 26 are arranged in the upper space 207 of the attachment space 206. Therefore, failures caused by adhesion of liquid or immersion in liquid are suppressed, as compared to the case where this configuration is not adopted. In addition, in the blood purification device 1, the power supply unit 24 and the electronic circuit unit 26, which have a larger mass than the tubes, etc., are located at the upper position, and the second group of components 34, which also has a larger mass than the tubes, etc., is located therebelow. Therefore, as compared to the case where this configuration is not adopted, a change in the position of the center of gravity before and after attachment is small and it is possible to ensure mobility and also suppress falling over.

When the user moves the blood purification device 1 by pushing on the back surface 201 side, since the RO unit 3 is attached to the back surface 201 side, the RO unit 3 is located on the user side and this makes it easier for the user to move the device, as compared to the case where this configuration is not adopted.

The blood purification device 1 is configured such that the carbon filter 32 and the reverse osmosis membrane 33 can be replaced by detaching the replacement cover 10b of the backside cover 10. This makes replacement easier than the case where replacement is only possible when all the covers on the back side are removed.

The blood purification device 1 has the liquid supply unit 28 capable of receiving the external dialysis water L₉ produced externally. Therefore, even in the event of a failure of the RO unit 3, the interruption of treatment can be reduced as compared to the case where this configuration is not adopted.

### Summary of the embodiment

Technical ideas understood from the embodiment will be described below citing the reference signs, etc., used for the embodiment. However, each reference sign, etc., described below is not intended to limit the constituent elements in the claims to the members, etc., specifically described in the embodiment.

### (First feature)

(1) A blood purification device 1 that purifies blood L₄ using dialysate L₃, the blood purification device 1 comprising: a first unit (the dialysis machine main body 2) that is a main body of the device; and a second unit (the RO unit 3) to be attached to the first unit (the dialysis machine main body 2), wherein the first unit (the dialysis machine main body 2) comprises a first group of components 27 requiring maintenance, and an attachment space 206 in which the first group of components 27 is exposed, wherein the attachment space 206 is open to the outside of the first unit (the dialysis machine main body 2) in a state that the second unit (the RO unit 3) is detached from the first unit (the dialysis machine main body 2), and wherein the attachment space 206 is closed by the second unit (the RO unit 3) in a state that the second unit (the RO unit 3) is attached to the first unit (the dialysis machine main body 2). In this blood purification device, the first group of components requiring maintenance becomes accessible by detaching the second unit from the first unit. Therefore, maintainability can be improved as compared to the case where this configuration is not adopted.

### (Second feature)

(2) The blood purification device 1 as defined by (1), wherein the first unit (the dialysis machine main body 2) comprises a dialysate production unit (the dialysate supply/discharge unit 23) that produces the dialysate L₃ by mixing dialysis water L₂ with dialysate concentrate (the undiluted B-solution L₆ and the undiluted A-solution L₇) and introduces and withdraws the dialysate L₃ to and from a blood purifier (the dialyzer 5) that purifies blood L₄ of a patient 9, and a first attachment portion 29, and the first group of components 27 requiring maintenance for at least one of production and introduction/withdrawal of the dialysate L₃ is exposed in the attachment space 206, and wherein the second unit (the RO unit 3) comprises a second attachment portion 300 to be attached to and detached from the first attachment portion 29 in a manner capable of closing the attachment space 206 when attaching the second unit (the RO unit 3) to the first unit (the dialysis machine main body 2) and exposing the attachment space 206 when detaching the second unit (the RO unit 3), a liquid supply port 37 through which external raw water L₁ is supplied, and a dialysis water production unit 31 to produce the dialysis water L₂ by removing an impurity from the raw water L₁ supplied through the liquid supply port 37, and the produced dialysis water L₂ is supplied to the first unit (the dialysis machine main body 2). In this blood purification device, the first group of components requiring maintenance is exposed in the attachment space. Therefore, as compared when not exposed, maintainability can be improved.

### (Third feature)

(3) The blood purification device 1 as defined by (2), wherein the second unit (the RO unit 3) comprises a base plate 30, wherein the base plate 30 comprises a first surface 301 facing the first unit (the dialysis machine main body 2) and a second surface 302 opposite to the first surface 301, and wherein a carbon filter 32 and a reverse osmosis membrane 33, which need to be replaced regularly, are arranged on the second surface 302. In this blood purification device, the carbon filter and the reverse osmosis membrane, which need to be replaced regularly, are arranged on the second surface. This makes replacement easy as compared to when arranged on the first surface.

### (Fourth feature)

(4) The blood purification device 1 as defined by (3), wherein a second group of components 34 requiring maintenance is arranged on the first surface 301. Maintainability of this blood purification device is high since the second group of components is located on the first surface when the second unit is detached.

### (Fifth feature)

(5) The blood purification device 1 as defined by (4), wherein the base plate 30 comprises, on the second surface 302, a first support portion 35 supporting the carbon filter 32, and a second support portion 36 supporting the reverse osmosis membrane 33, and wherein the first support portion 35 and the second support portion 36 are configured such that the carbon filter 32 and the reverse osmosis membrane 33 can be attached and detached using a thumbscrew 354 that does not require a tool. In this blood purification device, the carbon filter and the reverse osmosis membrane are attached through the thumbscrews and are thus detached and attached easily as compared to the case where this configuration is not adopted.

### (Sixth feature)

(6) The blood purification device 1 as defined by (5), wherein in the first unit (the dialysis machine main body 2), a power supply unit 24 and an electronic circuit unit 26 are arranged in an upper space 207 of the attachment space 206 and the first group of components 27 is exposed at an inner side in a lower portion serving as a maintenance space 208, and wherein when the second unit (the RO unit 3) is attached to the first unit (the dialysis machine main body 2), the second group of components 34 is located at an outer side of the maintenance space 208. In this blood purification device, the power supply unit and electronic circuit unit are located at the upper position. Therefore, failures caused by adhesion of liquid are less likely to occur as compared to when located at the lower position. In addition, in the blood purification device, the power supply unit and the electronic circuit unit, which have a larger mass than the tubes, etc., are located at the upper position, and the second group of components, which also has a larger mass than the tubes, etc., is located therebelow. Therefore, as compared to the case where this configuration is not adopted, a change in the position of the center of gravity before and after attachment is small and it is possible to ensure mobility and also suppress falling over.

### (Seventh feature)

(7) The blood purification device 1 as defined by (6), wherein the first unit (the dialysis machine main body 2) comprises a front surface 200 and a back surface 201 that is a surface opposite to the front surface 200, and when a main movement direction of the first unit (the dialysis machine main body 2) is a direction in which a user moves by pushing the back surface 201 side, the second unit (the RO unit 3) is attached to the first unit (the dialysis machine main body 2) with the first attachment portion 29 located on the back surface 201 side. In this blood purification device, the second unit is attached to the back surface. Therefore, the second unit is located on the user side and this makes it easier for the user to move the device as compared to the case where this configuration is not adopted.

### (Eighth feature)

(8) The blood purification device 1 as defined by (7), wherein the first unit (the dialysis machine main body 2) comprises a backside cover 10 that is located on the back surface 201 side and covers the attached second unit (the RO unit 3), and wherein the backside cover 10 comprises a replacement cover 10b that is removed to expose the carbon filter 32 and the reverse osmosis membrane 33 to allow replacement thereof. This blood purification device is configured such that the carbon filter and the reverse osmosis membrane can be replaced without entirely removing the backside cover. This makes replacement easier than the case where replacement is only possible when the backside cover is removed.

### (Ninth feature)

(9) The blood purification device 1 as defined by (8), wherein the first unit (the dialysis machine main body 2) comprises a liquid supply unit 28 that is located in the attachment space 206 and is capable of receiving dialysis water (the external dialysis water L₉) other than the dialysis water L₂ produced by the second unit (the RO unit 3). Even in the event that dialysis water is not supplied from the second unit, this blood purification device can use dialysis water produced externally. Therefore, even if the RO unit 3 fails, the interruption of treatment can be reduced as compared to the case where this configuration is not adopted.

### (Tenth feature)

(10) The blood purification device as defined by (1), wherein the second unit (the RO unit 3) comprises a base plate 30, wherein the base plate 30 comprises a first surface 301 attached to the first unit (the dialysis machine main body 2) and a second surface 302 opposite to the first surface 301, wherein a second group of components 34 requiring maintenance is arranged on the first surface 301, wherein the first unit (the dialysis machine main body 2) comprises a housing 20 to house the first group of components 27 thereinside, and the attachment space 206 in which the second unit (the RO unit 3) is attached, wherein the attachment space 206 comprises an opening 206a that is open to the outside of the housing 20 in a direction intersecting a gravity direction of the housing 20, an upper space 207, and a maintenance space 208 that is a lower space, wherein the maintenance space 208 comprises a first region 208a, and a second region 208b adjacent to the first region 208a and located at the inner side of the housing 20, wherein a power supply unit 24 is arranged in the upper space 207 of the attachment space 206, and wherein when the second unit (the RO unit 3) is attached to the first unit (the dialysis machine main body 2), the base plate 30 of the second unit (the RO unit 3) is attached to the housing 20 from outside the first region 208a so as to close the maintenance space 208, the second group of components 34 is positioned below the power supply unit 24 in the first region 208a, and the first group of components 27 is arranged in the second region 208b so as to face the second group of components 34. In this blood purification device, the first group of components and the second group of components are exposed when the second unit is detached from the first unit. Therefore, as compared to the case where this configuration is not adopted, maintenance on the first group of components and the second group of components can be easily performed by simply detaching the second unit from the first unit.

### (Eleventh feature)

(11) The blood purification device as defined by (10), wherein the power supply unit 24 is positioned above the second group of components 34 in the gravity direction so that a change in a position of the center of gravity of the first unit (the dialysis machine main body 2) before and after attaching the second unit (the RO unit 3) to the first unit (the dialysis machine main body 2) is small. The change in the position of the center of gravity of this blood purification device is small even after the second unit is attached to the first unit. Therefore, it is possible to suppress falling over regardless of with or without the second unit, as compared to the case where the center of gravity changes significantly.

### (Twelfth feature)

(12) The blood purification device as defined by (10), wherein a plurality of casters 209 to move the first unit (the dialysis machine main body 2) are provide on a lower surface of the housing 20, and wherein the change in the position of the center of gravity of the first unit (the dialysis machine main body 2) before and after attaching the second unit (the RO unit 3) to the first unit (the dialysis machine main body 2) is small in a movement direction of the casters 209. This blood purification device can ensure the mobility of the first unit and also suppress falling over, regardless of with or without the second unit.

Although the embodiment of the invention has been described, this embodiment is merely an example and the invention according to claims is not to be limited thereto. These new embodiments may be implemented in various other forms, and various omissions, substitutions and changes, etc., can be made without departing from the gist of the invention. In addition, not all combinations of the features described in the embodiment are necessary to solve the problem of the invention. Further, the embodiment is included within the scope and gist of the invention and also within the invention described in the claims and the range of equivalency.

### REFERENCE SIGNS LIST

1: blood purification device, 2: dialysis machine main body, 3: RO unit, 5: dialyzer, 10: backside cover, 10a: upper cover, 10b: replacement cover, 12: handle, 23: dialysate supply/discharge unit, 24: power supply unit, 26: electronic circuit unit, 27: first group of components, 28: liquid supply unit, 29: first attachment portion, 30: base plate, 31: dialysis water production unit, 32: carbon filter, 33: reverse osmosis membrane, 34: second group of components, 35: first support portion, 36: second support portion, 206: attachment space, 206a: opening, 207: upper space, 208: maintenance space, 208a: first region, 208b: second region, 300: second attachment portion, 301: first surface, 302: second surface, L₁: raw water, L₂: dialysis water, L₃: dialysate, L₄: blood, L₅: purified blood, L₆: B-solution, L₇: A-solution, L₈: dialysate effluent, L₉: external dialysis water, L₁₀: cleaning water, :L₁₁: disinfectant

## Claims

1. A blood purification device that purifies blood using dialysate, the blood purification device comprising:
a first unit that is a main body of the device; and
a second unit to be attached to the first unit,
wherein the first unit comprises a first group of components requiring maintenance, and an attachment space in which the first group of components is exposed,
wherein the attachment space is open to the outside of the first unit in a state that the second unit is detached from the first unit, and
wherein the attachment space is closed by the second unit in a state that the second unit is attached to the first unit.

2. The blood purification device according to claim 1, wherein the first unit comprises a dialysate production unit that produces the dialysate by mixing dialysis water with dialysate concentrate and introduces and withdraws the dialysate to and from a blood purifier that purifies blood of a patient, and a first attachment portion, and the first group of components requiring maintenance for at least one of production and introduction/withdrawal of the dialysate is exposed in the attachment space, and wherein the second unit comprises a second attachment portion to be attached to and detached from the first attachment portion in a manner capable of closing the attachment space when attaching the second unit to the first unit and exposing the attachment space when detaching the second unit, a liquid supply port through which external raw water is supplied, and a dialysis water production unit to produce the dialysis water by removing an impurity from the raw water supplied through the liquid supply port, and the produced dialysis water is supplied to the first unit.

3. The blood purification device according to claim 2, wherein the second unit comprises a base plate, wherein the base plate comprises a first surface facing the first unit and a second surface opposite to the first surface, and wherein a carbon filter and a reverse osmosis membrane, which need to be replaced regularly, are arranged on the second surface.

4. The blood purification device according to claim 3, wherein a second group of components requiring maintenance is arranged on the first surface.

5. The blood purification device according to claim 4, wherein the base plate comprises, on the second surface, a first support portion supporting the carbon filter, and a second support portion supporting the reverse osmosis membrane, and wherein the first support portion and the second support portion are configured such that the carbon filter and the reverse osmosis membrane can be attached and detached using a thumbscrew that does not require a tool.

6. The blood purification device according to claim 5, wherein in the first unit, a power supply unit and an electronic circuit unit are arranged in an opening upper portion of the attachment space and the first group of components is exposed at an inner side in a lower portion serving as a maintenance space, and wherein when the second unit is attached to the first unit, the second group of components is located at an outer side of the maintenance space.

7. The blood purification device according to claim 6, wherein the first unit comprises a front surface and a back surface that is a surface opposite to the front surface, and when a main movement direction of the first unit is a direction in which a user moves by pushing the back surface side, the second unit is attached to the first unit with the first attachment portion located on the back surface side.

8. The blood purification device according to claim 7, wherein the first unit comprises a backside cover that is located on the back surface side and covers the attached second unit, and wherein the backside cover comprises a replacement cover that is removed to expose the carbon filter and the reverse osmosis membrane to allow replacement thereof.

9. The blood purification device according to claim 8, wherein the first unit comprises a liquid supply unit that is located in the attachment space and is capable of receiving dialysis water other than the dialysis water produced by the second unit.

10. The blood purification device according to claim 1, wherein the second unit comprises a base plate, wherein the base plate comprises a first surface attached to the first unit and a second surface opposite to the first surface, wherein a second group of components requiring maintenance is arranged on the first surface, wherein the first unit comprises a housing to house the first group of components thereinside, and the attachment space in which the second unit is attached, wherein the attachment space comprises an opening that is open to the outside of the housing in a direction intersecting a gravity direction of the housing, an upper space, and a maintenance space that is a lower space, wherein the maintenance space comprises a first region, and a second region adjacent to the first region and located at the inner side of the housing, wherein a power supply unit is arranged in the upper space of the attachment space, and wherein when the second unit is attached to the first unit, the base plate of the second unit is attached to the housing from outside the first region so as to close the maintenance space, the second group of components is positioned below the power supply unit in the first region, and the first group of components is arranged in the second region so as to face the second group of components.

11. The blood purification device according to claim 10, wherein the power supply unit is positioned above the second group of components in the gravity direction so that a change in a position of the center of gravity of the first unit before and after attaching the second unit to the first unit is small.

12. The blood purification device according to claim 10, wherein a plurality of casters to move the first unit are provide on a lower surface of the housing, and wherein the change in the position of the center of gravity of the first unit before and after attaching the second unit to the first unit is small in a movement direction of the casters.
